# EUROPEAN PATENT APPLICATION

(11) **EP 2 193 841 A1**
(43) Date of publication of application: **09.06.2010**
(21) Application number: 08021305.1
(22) Date of filing: 08.12.2008
(51) Int. Cl.: B01J 23/78, B01J 35/10, C07C 1/04, C10G 2/00, B01J 23/745, B01J 37/03, B01J 27/02, B01J 27/185, B01J 27/043, B01J 37/18

(54) **Hydrocarbon synthesis catalyst and process with improved selectivity to short chain products**

(71) Applicant: Sasol Technology (Proprietary) Limited, 2196 Rosebank (ZA)
(72) Inventor: Crous, Reinier, SW5 Ext. 2 1911 (ZA); Bromfield, Tracey, Carolyn, Vanderbijlpark 1911 (ZA); Booyens, Sharon, Cardiff CF10 4RF (GB)
(74) Representative: Kador & Partner

(57) **Abstract**

The present invention relates to a hydrocarbon synthesis catalyst comprising in its unreduced form
a) Fe as catalytically active metal,
b) an alkali metal and/or alkaline-earth metal in an alkali metal- and/or alkaline-earth metal-containing promoter, the alkali metal being present in an amount of from 0.1 to 1.0 g/100 g Fe,
c) and a further promoter comprising, or consisting of, one or more element(s) selected from the group of boron, silicon, germanium, nitrogen, phosphorus, arsenic, antimony, sulphur, selenium and tellurium, the element(s) being present in an amount of from 0.03 to 0.2 g/100 g Fe, and

wherein the catalyst in its unreduced form has a surface area of less than 50 m²/g, to a hydrocarbon synthesis process which is operated in the present of such a catalyst and to the use of such a catalyst in a hydrocarbon synthesis process.

## Description

This invention relates to a hydrocarbon synthesis catalyst comprising Fe as catalytically active metal, to a Fischer-Tropsch process in which this catalyst is used and to the use of the catalyst in a Fischer-Tropsch process.

Hydrocarbon synthesis by the Fischer-Tropsch process is known to produce a variety of products including paraffins, olefins, and oxygenates with different carbon chain lengths and isomers. One of the most serious drawbacks of the Fischer-Tropsch synthesis is that the product spectrum usually obtained is broad as predicted by the Schulz-Flory product distribution model.

The chemical industry uses as feedstock mainly short chain olefins, particularly the C₂-C₄ olefins. These olefins are normally derived from various crude oil sources. A synthetic route based on the Fischer-Tropsch technology would be highly desirable from an economic point of view as the reliance on crude oil would be eliminated.

It is known in the art that to some extent selectivity is a function of conversion, and that selectivity to lower molecular weight hydrocarbons can be enhanced by operating at process conditions for low conversion of CO. Preferred methods for lowering the conversion include increasing the space velocity and lowering the temperature. However, low conversions of valuable feed gas are undesirable for commercial applications because of low yields for the desired products.

It is also known in the art that methane production can be suppressed by dramatically lowering the H₂:CO ratio of the feed gas. This has the detrimental effect of increasing the selectivity for higher molecular weight hydrocarbons.

A further approach to enhance the yield of C₂-C₄ olefins in a Fischer-Tropsch synthesis without simultaneously producing large quantities of the corresponding C₂-C₄ paraffins was the development of new catalysts which selectively produce the short chain olefins and simultaneously minimize the production of short chain paraffins, especially methane.

Many catalyst examples in the art relate to improvements in the selectivity to a desirable product range of C₂-C₄ compounds. For example, in US 4,199,522 and US 4,151,190 a method is disclosed for increasing the selectivity to C₂-C₄ olefins by using a catalyst comprising at least one member of the group of metals, oxides or sulfides of molybdenum, tungsten, rhenium, ruthenium, nickel, palladium, rhodium, osmium, iridium, and platinum, and at least one member of the group of hydroxides, oxides, or salts of alkaline or alkaline earth metals.

In US 4,539,334 the addition of phosphorus (in the form of a volatile phosphorous precursor) to a conventional Fischer-Tropsch catalyst is claimed to improve the selectivity of said catalyst to C₂-C₄ olefins. However, these results were obtained at relatively low conversions of CO at a H₂:CO ratio of ≤ 1.

In a study by Wu et al. (See Fuel, [2004], 83, pp. 205-212) a precipitated iron Fischer-Tropsch catalyst containing sulphur was prepared by using ferrous sulphate as precursor. It was shown that small amounts of sulphur may promote the catalyst by increasing activity and improving the heavier hydrocarbon selectivity.

More recently, Zhang et al. (see J. Nat. Gas Chem., [2007], 16, 377-381) studied the influence of several anions on the performance of Fe-based Fischer-Tropsch catalysts. The catalyst used in this investigation was of the composition Fe:Mn:K:activated carbon = 25:20:5:50 by weight. From this study it was revealed that the addition of 500 ppm of anions lowered the catalytic activity of the catalyst for Fischer-Tropsch synthesis as well as the yield of light olefins. The selectivity for methane was also lowered by the presence of the anions.

Finally, in US 6,653,357 a method for conducting a high temperature Fischer-Tropsch synthesis is disclosed with which the selectivity profile of the lower olefins is improved by injecting a promoter-carrying compound directly into the reactor medium.

However, in spite of the various approaches to obtain an improved yield of short chain products described above there is still the need for improved Fischer-Tropsch catalysts tailored to produce a high amount of C₂-C₄ products, especially C₂-C₄ olefins, while keeping the methane formation at a low level.

It is thus the object of the present invention to provide a hydrocarbon synthesis catalyst has an improved selectivity for C₂-C₄ products, especially C₂-C₄ olefins, with simultaneous suppression of excess methane formation. Simultaneously, the catalyst should have high activity and should allow for high CO conversion rates.

Surprisingly, it has now been found that such a catalyst can be provided if it contains Fe as catalytically active metal, an alkali and/or alkaline earth metal promoter and, in addition, a further promoter which is selected from the group of non-metals and semi-metals (Metalloids) found in groups 13 to 16 of the Periodic Table of the Elements.

The present invention therefore provides a hydrocarbon synthesis, especially a Fischer-Tropsch synthesis, catalyst comprising in its unreduced form
a) Fe as catalytically active metal,
b) an alkali metal and/or alkaline-earth metal in an alkali metal- and/or alkaline-earth metal-containing promoter, the alkali metal and/or alkaline-earth metal being present in an amount of from 0.1 to 1.0 g/100 g Fe,
c) and a further promoter comprising, or consisting of, one or more element(s) selected from the group of boron, silicon, germanium, nitrogen, phosphorus, arsenic, antimony, sulphur, selenium and tellurium, the element(s) being present in an amount of from 0.03 to 0.2 g/100 g Fe, and
wherein the catalyst in its unreduced form has a surface area of less than 50 m²/g.

The catalyst allows for a dramatic increase in selectivity to light hydrocarbons with a simultaneous suppression of the expected increase in methane formation when used in hydrocarbon synthesis, especially Fischer-Tropsch synthesis, in which a feed gas composed of H₂ and at least one carbon oxide is converted at elevated temperature and pressure. Furthermore, the light fraction of the obtained hydrocarbons consist mostly of valuable olefins and alcohols with minimal paraffin formation. Still further, the catalyst shows a good activity and synthesis gas conversion.

Finally, due to its surface area, the catalyst is especially well suited for high temperature hydrocarbon synthesis processes.

The catalyst in its unreduced form preferably has a surface area of 40 m²/g or less, and more preferably of 35 m²/g or less.

Furthermore, the catalyst in its unreduced form preferably has a surface area of 10 m²/g or more, more preferably of 25 m²/g or more.

The amount of Fe in the unreduced form of the catalyst preferably is from 60 to 80 wt%, more preferably from 65 to 75 wt.%. The balance in the unreduced form of the catalyst, excluding Fe and promoters, may be made up of oxygen, so that Fe is present in the catalyst in the form of iron oxides, preferably in the form of hematite.

The catalyst in its reduced form preferably has a surface area of not more than 30 m²/g, more preferably of not more than 20 m²/g, and most preferably of not more than 10 m²/g. Usually, the surface area of the catalyst in its reduced form is not less than 1 m²/g.

The amount of Fe in the reduced form of the catalyst preferably is from 50 to 99.0 wt%, more preferably from 60 to 90 wt.%. The balance in the reduced form of the catalyst, excluding Fe and promoters, may be made up of carbon in the form of carbide.

Preferably, Fe is the only catalytically active metal in the catalyst.

The catalyst preferably comprises, more preferably is, a precipitated catalyst, but it may also comprise or be a fused catalyst. The precipitated catalyst preferably comprises a precipitated catalyst as described in WO 01/97968 A2.

The alkali metal- and/or alkaline-earth metal-containing promoter may contain more than one type of alkali metal- and/or alkaline-earth metal. The alkali metal- and/or alkaline-earth metal-containing promoter of the catalyst usually is in the form of an alkali metal oxide and/or alkaline-earth metal oxide.

Preferably, the alkali metal in the alkali metal-containing promoter is Na or K, and hence the alkali metal-containing promoter preferably comprises potassium oxide or sodium oxide.

Preferably, the alkaline-earth metal in the alkaline-earth metal-containing promoter is Ca or Mg.

Preferably, the alkali metal and/or alkaline-earth metal in the alkali metal- and/or alkaline-earth metal-containing promoter is/are present in the unreduced form of the catalyst in an amount of from 0.1 to 0.6 g/100 g Fe.

The alkali metal- and/or alkaline-earth metal-containing promoter can be added to the iron by means of various methods, such as impregnation of the iron with the alkali metal- or alkaline-earth metal-containing promoter, co-precipitating the alkali metal- or alkaline-earth metal-containing promoter, fusing the iron and the alkali metal- or alkaline-earth metal-containing promoter, addition of the alkali metal- or alkaline-earth metal-containing promoter directly to the reaction zone, etc.

In addition to the alkali metal- and/or alkaline-earth metal-containing promoter, the catalyst contains at least one further promoter which contains, or consists of, one or more element(s) selected from the group of boron, silicon, germanium, nitrogen, phosphorus, arsenic, antimony, sulphur, selenium and tellurium.

In an embodiment of the invention the alkali metal- or alkaline-earth metal-containing promoter and the at least one further promoter are chemically bound to another, in the form of a single compound. Preferably in this embodiment, the single compound is NaS.

In one preferred embodiment of the invention the further promoter which contains, or consists of, one or more element(s) selected from the group of boron, silicon, germanium, nitrogen, phosphorus, arsenic, antimony, sulphur, selenium and tellurium comprises, or consists of, one or more element(s) selected from the group of boron, phosphorus, antimony and sulphur, and most preferably comprises, or consists of, sulphur.

In case the further promoter consists of sulphur, the precursor may be ammonium sulphate, but other sulphur precursors may also be used.

Preferably, the element(s) in the further promoter comprising one or more element(s) selected from the group of boron, silicon, germanium, nitrogen, phosphorus, arsenic, antimony, sulphur, selenium and tellurium is(are) present in the catalyst in an amount of from 0.05 to 0.15 g/100 g Fe.

Preferably, in the unreduced form of the catalyst according to invention the weight ratio between the alkali metal and/or alkaline-earth metal in the alkali metal- and/or alkaline-earth metal-containing promoter and the element(s) in the further promoter comprising one or more element(s) selected from the group of boron, silicon, germanium, nitrogen, phosphorus, arsenic, antimony, sulphur, selenium and tellurium is from 0.5:1 to 33:1, more preferably is from 0.8:1 to 20:1.

In addition to the promoters described above, the catalyst may also contain other promoters. Certain promoters, for example Al, Ti or Cr, can be added as promoters to the catalyst. Binders, such as silica or alumina, may also be added in case of a spray-dried catalyst.

If the process is to be performed in a fluidised bed reactor, the final catalyst may be produced by means of a variety of known methods in order to obtain particles with acceptable fluidisation properties, such as crushing, spray-drying, etc. In order to obtain a particle size distribution suitable for fluidisation, the catalyst may be classified by means of known methods, such as sieving, cyclone classification etc.

The unreduced catalyst preferably is in a particulate form in which more than 90 wt.% of the particles have a particle size in the range of 10 to 250 µm, preferably have a particle size in the range of 20 to 180 µm and most preferably have a particle size in the range of 38 to 150 µm.

The catalyst may be brought into the desired particulate form by crushing and sieving.

Preferably, the catalyst is an unsupported catalyst.

The present invention furthermore relates to a hydrocarbon synthesis process, especially a Fischer-Tropsch process, which is operated in the presence of a catalyst according to any of the above described embodiments, wherein the catalyst is in its reduced form.

It will be appreciated that if the catalyst is still in its unreduced form, it is reduced prior to or upon initiation of the hydrocarbon synthesis, especially Fischer-Tropsch, reaction, to provide the catalyst in its reduced form.

The reduction of the catalyst may be carried out in-situ. In this context "in-situ" denotes that the catalyst is placed in the reaction zone of the hydrocarbon synthesis process, e.g. a fluidized bed reactor or a fixed fluidized bed reactor, and reduced before the hydrocarbon synthesis reaction conditions are applied to said reaction zone.

Preferably, the reduction is carried out using H₂ and/or CO as reducing gas, more preferably H₂ is used as reducing gas.

Preferably the reduction is carried out at a temperature of at least 200 °C, more preferably of at least 275 °C, still more preferably of at least 300 °C, still more preferably of at least 350 °C, and most preferably of at least 380 °C.

The reduction is preferably carried out at a temperature of not more than 800 °C, more preferably of not more than 650 °C and most preferably of not more than 500 °C.

Preferably, the reduction is carried out for at least 8 hours, more preferably for at least 11 hours and most preferably for at least 14 hours.

The reduction is preferably carried out for at most 24 hours, more preferably for at most 21 hours and most preferably for at most 18 hours.

Preferably, the reduction is carried out at a pressure of at least 1.0 MPa, more preferably of at least 1.3 MPa and most preferably of at least 1.6 MPa.

The reduction is preferably carried out at a pressure of not more than 3.0 MPa, more preferably of not more than 2.7 MPa and most preferably of not more than 2.4 MPa.

During the reduction step, at least some of the Fe is reduced from e.g. an Fe-oxide to Fe in the zero oxidation state. Preferably, at least 75 wt.% of the Fe is reduced to Fe in the zero oxidation state.

It will be appreciated that the concentrations of the alkali metal and/or alkaline earth metal in the alkali metal- and/or alkaline-earth metal-containing promoter and the element(s) in the further promoter comprising one or more element(s) selected from the group of boron, silicon, germanium, nitrogen, phosphorus, arsenic, antimony, sulphur, selenium and tellurium are not changed during the reduction step, as these are expressed as g/100g Fe.

Preferably, the hydrocarbon synthesis process is a high temperature process, more preferably is a high temperature Fischer-Tropsch process.

The high temperature hydrocarbon synthesis process is usually carried out at a temperature of 250 °C or higher, more preferably of 280 °C or higher, and most preferably of 300 °C or higher.

Preferably, the process is carried out at a temperature of 450 °C or lower, more preferably of 400 °C or lower, and most preferably of 360 °C or lower.

The total pressure in the reaction zone preferably is 5 bar or higher, more preferably is 10 bar or higher, and most preferably is 15 bar or higher.

Preferably, the total pressure is 60 bar or lower, more preferably is 50 bar or lower, still more preferably is 40 bar or lower, and most preferably is 30 bar or lower.

The high temperature hydrocarbon synthesis process can be described as a two-phase process. At the temperatures and pressures used for this process both reactants and the products are in the gas phase in the reaction zone, and the catalyst, which is a solid, forms the second phase.

The applicant has further observed that under the preferred high temperature Fischer Tropsch reaction conditions described hereinbefore, the catalyst produces remarkably low levels of free carbon. High levels of free carbon formation is a known disadvantage associated with commercial high temperature Fischer Tropsch processes, which leads to carbon deposition on the catalyst surface and therefore deactivation of the catalyst. As a result, commercial processes employ fluidized bed reactors to avoid blocking of the catalyst bed.

In a preferred embodiment of this invention, the hydrocarbon synthesis process, preferably the Fischer Tropsch process is conducted in a stationary bed reactor.

The stationary bed reactor may define a zone wherein the catalyst is held stationary within the reactor. The zone may be defined by a first and second screen, carrying the catalyst, wherein the apertures of the screens are too small for the catalyst particles to pass through.

The stationary reactor may be a fixed bed reactor or a fixed-fluidised bed reactor, but preferably, the stationary bed reactor is a fixed bed reactor.

Preferably, the feed gas in the process comprises at least one carbon oxide, more preferably comprises CO. The carbon oxide may comprise a mixture of CO and CO₂.

Furthermore, the feed gas preferably comprises H₂.

It is typical for the Fischer-Tropsch process that the feed gas both contains H₂ and carbon monoxide.

Preferably, the H₂:carbon oxide molar ratio may be as low as possible at or above the ratio of 2. However, it may be higher than 2:1, e.g. 2.5, and even as high as 3:1, or even higher than 3:1.

The H₂ and carbon oxide feed is known as synthesis feed gas and it may also include other components, such as water vapour, Ar, CH₄, light hydrocarbons, etc.

The invention also relates to the use of a catalyst in any of the above described embodiments in a hydrocarbon synthesis process, especially in a Fischer-Tropsch process.

The invention will now be described by means of the following non-limiting examples.

### Examples

### 1. Methods

### 1.1 Surface Area

Surface Area measurements were done using a Micromeritics Tristar 3000. Samples were degassed under dynamic nitrogen flow at 200 °C for a minimum time of 12 hours. Thereafter 250 - 300 mg of sample were accurately weighed out and loaded into 3/8 inch tubes onto the instrument. Sample tubes were immersed in a liquid nitrogen bath and evacuated. Leak tests were conducted, where after a total of eight relative pressure points were measured in a range from 0.08 to 0.98. Measured parameters were used by the instrument software to calculate the surface area.

### 1.2 Particle Size

The Particle size of the catalyst particles is determined using fine test sieves fitted with stainless steel wire cloth meeting ASTM specification E-11.

### 1.3 Catalyst Preparation Procedure

For the reverse precipitation of iron, up to 100 ml 25% (v/v) NH₄OH solution was added drop-wise, whilst stirring with an overhead stirrer, to 400 ml of 1 M aqueous solution of Fe(NO₃)₃·9 H₂O (161.6 g) until a pH of 7 at room temperature (25 °C) was reached. Thereafter, Na₂CO₃ and the selected non-metal precursor, if present, were added in the appropriate amounts to the precipitation mixture. The resultant slurry was then dried in a fan-oven overnight (approximately 16 hours) at 150 °C, and then calcined in air at 350 °C for 4 hours. Finally, the catalyst was crushed and screened to a particle size range of 38-150 µm. This was achieved by screening the catalyst over a 38 µm sieve and discarding the <38 µm fraction, followed by screening over a 150 µm sieve and discarding the >150 µm fraction using fine test sieves fitted with stainless steel wire cloth meeting ASTM specification E-11.

### 1.4 Catalyst Testing Procedure

25 mg calcined catalyst was loaded into a micro fixed bed reactor and reduced in situ under hydrogen at 420 °C for 16 hours at 20 bar. Thereafter, synthesis gas was introduced at a flow rate of 13 liters (n) per g catalyst per hour (H₂ = 57 volume %, CO = 14 volume %, CO₂ = 11 volume %) at 20 bar total pressure and at a temperature of 330 °C. Analysis of hydrocarbon products was performed using GC-FID, and permanent gas analysis was done by GC-TCD.

### 1.5 Selectivity

All selectivities are expressed as carbon-atom % selectivity and are not normalized. CO₂ formation is excluded in selectivity calculations.

### 2. Experiments

### Comparative Example 1:

Following the general catalyst preparation procedure described above a catalyst with a composition of 0.295 g Na per 100 g Fe was prepared and tested in a micro fixed bed reactor as described in the general catalyst testing procedure above. The synthesis results are summarized in Tables 1, 2, 3 and 4.

### Example 2:

Following the general catalyst preparation procedure described above a catalyst with a composition of 0.591 g Na per 100 g Fe and 0.12 g S per 100 g Fe was prepared and tested in a micro fixed bed reactor as described in the general catalyst testing procedure above. The sulphur precursor used was ammonium sulphate. The synthesis results are summarized in Table 1 and compared to Comparative Example 1.

**Table 1:**

| | Comparative Example 1† 0.295 g Na / 100 g Fe | Example 2* 0.591 g Na / 0.12 g S / 100 g Fe |
|---|---|---|
| CO+H₂ conversion | 54 % | 41 % |
| CO+CO₂ conversion | 50 % | 40 % |
| CH₄ selectivity | 9 % | 9 % |
| C₂-C₄ selectivity | 24 % | 52 % |
| C₂-C₈ selectivity | 52 % | 76 % |
| C₅₊ selectivity | 58 % | 34 % |
| % olefins in C₂-C₄ fractions | 80 % | 75 % |
| % alcohols in C₂-C₄ fractions | 12 % | 15 % |
| Surface area (unreduced catalyst) | 32.8 m²/g | 31.7 m²/g |

| | | |
|---|---|---|
| † estimated mass balance: 95 %; * estimated mass balance: 97 % | | |

### Example 3:

Following the general catalyst preparation procedure described above a catalyst with a composition of 0.31 g Na per 100 g Fe and 0.08 g P per 100 g Fe was prepared and tested in a micro fixed bed reactor as described in the general catalyst testing procedure above. The phosphorous precursor used was ammonium phosphate dibasic. The synthesis results are summarized in Table 2 and compared to Comparative Example 1.

**Table 2:**

| | Comparative Example 1† 0.295 g Na / 100 g Fe | Example 3* 0.31 g Na / 0.08 g P / 100 g Fe |
|---|---|---|
| CO+H₂ conversion | 54 % | 44 % |
| CO+CO₂ conversion | 50 % | 41 % |
| CH₄ selectivity | 9 % | 10 % |
| C₂-C₄ selectivity | 24 % | 37 % |
| C₂-C₈ selectivity | 52 % | 62 % |
| C₅₊ selectivity | 58 % | 45 % |
| % olefins in C₂-C₄ fractions | 80 % | 80 % |
| % alcohols in C₂-C₄ fractions | 12 % | 8 % |

| | | |
|---|---|---|
| † estimated mass balance: 95 %; * estimated mass balance: 93 % | | |

### Example 4:

Following the general catalyst preparation procedure described above a catalyst with a composition of 0.0369 g Na per 100 g Fe and 0.05 g B per 100 g Fe was prepared and tested in a micro fixed bed reactor as described in the general catalyst testing procedure methodology above. The boron precursor used was ammonium biborate tetrahydrate. The synthesis results are summarized in Table 3 and compared to Comparative Example 1.

**Table 3:**

| | Comparative Example 1† 0.295 g Na / 100 g Fe | Example 4* 0.0369 g Na / 0.05 g B / 100 g Fe |
|---|---|---|
| CO+H₂ conversion | 54 % | 47 % |
| CO+CO₂ conversion | 50 % | 43 % |
| CH₄ selectivity | 9 % | 9 % |
| C₂-C₄ selectivity | 24 % | 37 % |
| C₂-C₈ selectivity | 52 % | 67 % |
| C₅₊ selectivity | 58 % | 49 % |
| % olefins in C₂-C₄ fractions | 80 % | 82 % |
| % alcohols in C₂-C₄ fractions | 12 % | 6 % |

| | | |
|---|---|---|
| † estimated mass balance: 95 %; * estimated mass balance: 95 % | | |

### Example 5:

Following the general catalyst preparation procedure described above a catalyst with a composition of 0.591 g Na per 100 g Fe and 0.1 g Sb per 100 g Fe was prepared and tested in a micro fixed bed reactor as described in the general catalyst testing methodology above. The antimony precursor used was antimony acetate. The synthesis results are summarized in Table 4 and compared to Comparative Example 1.

**Table 4:**

| | Comparative Example 1† 0.295g Na / 100g Fe | Example 5* 0.591 g Na / 0.1 g Sb / 100g Fe* |
|---|---|---|
| CO+H₂ conversion | 54 % | 41 % |
| CO+CO₂ conversion | 50 % | 39 % |
| CH₄ selectivity | 9 % | 7 % |
| C₂-C₄ selectivity | 24 % | 38 % |
| C₂-C₈ selectivity | 52 % | 67 % |
| C₅₊ selectivity | 58 % | 49 % |
| % olefins in C₂-C₄ fractions | 80 % | 73 % |
| % alcohols in C₂-C₄ fractions | 12 % | 14 % |

| | | |
|---|---|---|
| † estimated mass balance: 95 %; * estimated mass balance: 94 % | | |

### Discussion of the results in Tables 1 to 4:

Surprisingly, when comparing the synthesis performance of the catalyst of Comparative Example 1 with the catalysts promoted by a series of non-metals in combination with alkali metal (Examples 2 to 5), it will be noted that the methane selectivity remained constant while a dramatic increase in selectivity for light hydrocarbons (C₂-C₄ hydrocarbon fraction) was observed.

## Claims

1. A hydrocarbon synthesis catalyst comprising in its unreduced form
a) Fe as catalytically active metal,
b) an alkali metal and/or alkaline-earth metal in an alkali metal- and/or alkaline-earth metal-containing promoter, the alkali metal and/or alkaline-earth metal being present in an amount of from 0.1 to 1.0 g/100 g Fe,
c) and a further promoter comprising one or more element(s) selected from the group of boron, silicon, germanium, nitrogen, phosphorus, arsenic, antimony, sulphur, selenium and tellurium, the element(s) being present in an amount of from 0.03 to 0.2 g/100 g Fe, and
wherein the catalyst in its unreduced form has a surface area of less than 50 m²/g.

2. Catalyst according to claim 1 wherein the amount of Fe in the reduced form of the catalyst is from 50 to 99.0 wt%.

3. Catalyst according to any of the preceding claims wherein the alkali metal and/or alkaline-earth metal in the alkali metal- and/or alkaline-earth metal-containing promoter is/are present in the catalyst in an amount of from 0.1 to 0.6 g/100 g Fe.

4. Catalyst according to any of the preceding claims wherein the alkali metal and/or alkaline-earth metal in the alkali metal- and/or alkaline-earth metal-containing promoter is Na or K.

5. Catalyst according to any of the preceding claims wherein the one or more element(s) in the further promoter comprising one or more element(s) selected from the group of boron, silicon, germanium, nitrogen, phosphorus, arsenic, antimony, sulphur, selenium and tellurium is(are) present in the catalyst in an amount of from 0.05 to 0.15 g/100 g Fe.

6. Catalyst according to any of the preceding claims wherein the further promoter comprises sulphur.

7. Catalyst according to any of the preceding claims wherein in the catalyst the weight ratio between the alkali metal and/or alkaline-earth metal in the alkali metal and/or alkaline-earth metal-containing promoter and the element(s) in the further promoter comprising one or more element(s) selected from the group of boron, silicon, germanium, nitrogen, phosphorus, arsenic, antimony, sulphur, selenium and tellurium is from 0.5:1 to 50:1.

8. Catalyst according to any of the preceding claims wherein the catalyst in its unreduced form has a surface area of from 10 to 40 m²/g.

9. Catalyst according to any of the preceding claims wherein the catalyst is in a particulate form in which at least 90 wt.% of the particles have a particle size of from 10 to 250 µm.

10. A hydrocarbon synthesis process which is operated in the presence of a catalyst according to any of the preceding claims, wherein the catalyst is in its reduced form.

11. Process according to claim 10 which is a Fischer-Tropsch process.

12. Process according to claim 10 or 11 which is operated at a temperature of 250 °C or higher.

13. Process according to any of claims 10 to 12 wherein the feed gas comprises H₂ and carbon oxide and the H₂:carbon oxide molar feed ratio is greater than 2:1.

14. Process according to any of claims 13 to 19 which is operated at a pressure of from 5 to 60 bar.

15. Use of a catalyst according to any of claims 1 to 9 in a hydrocarbon synthesis process.
